# EUROPÄISCHE PATENTANMELDUNG

(11) **EP 3 396 572 A1**
(43) Veröffentlichungstag der Anmeldung: **31.10.2018**
(21) Anmeldenummer: 17167787.5
(22) Anmeldetag: 24.04.2017
(51) Int. Cl.: G06F 19/00, A61B 6/00, A61B 6/03, A61B 5/055, G01R 33/54

(54) **VERFAHREN ZUM BETRIEB EINER BILDAUFNAHMEEINRICHTUNG, BILDAUFNAHMEEINRICHTUNG, COMPUTERPROGRAMM UND ELEKTRONISCH LESBARER DATENTRÄGER**

(71) Anmelder: Siemens Healthcare GmbH, 91052 Erlangen (DE)
(72) Erfinder: Meyer, Stefan, 91094 Langensendelbach (DE); Schor, Stefan, 91054 Erlangen (DE)

(57) **Zusammenfassung**

Verfahren zum Betrieb einer Bildaufnahmeeinrichtung, insbesondere einer Magnetresonanzeinrichtung, welche eine Speichereinrichtung für eine Mehrzahl von Aufnahmeparametern der Bildaufnahmeeinrichtung enthält und welche einen Aufnahmevorgang definierende Aufnahmeprotokolle aufweist, wobei bei einer den möglichen Einstellungsraum für Aufnahmeparameter verändernden Aktualisierung wenigstens einer Software- und/oder Hardwarekomponente der Bildaufnahmeeinrichtung auf eine aktuelle Ausstattungsversion und/oder bei Eingang eines nicht für die aktuelle Ausstattungsversion definierten Aufnahmeprotokolls ein Regelsatz auf alle nicht für die aktuelle Ausstattungsversion definierten Protokolle angewendet wird, welcher wenigstens eine Anpassungsregel für wenigstens einen Aufnahmeparameter umfasst, wobei die Anpassungsregel bei Anwendbarkeit wenigstens ein Qualitätsmaß für mittels des Aufnahmeprotokolls, auf das die Anpassungsregel angewendet wird, aufgenommene Bilddaten erhöht.

## Beschreibung

Die Erfindung betrifft ein Verfahren zum Betrieb einer Bildaufnahmeeinrichtung, insbesondere einer Magnetresonanzeinrichtung, welche eine Speichereinrichtung für eine Mehrzahl von Aufnahmeparameter der Bildaufnahmeeinrichtung enthaltenden, einen Aufnahmevorgang definierenden Aufnahmeprotokollen aufweist. Daneben betrifft die Erfindung eine Bildaufnahmeeinrichtung, ein Computerprogramm und einen elektronisch lesbaren Datenträger.

Bildaufnahmeeinrichtungen unterschiedlicher Modalitäten werden oft im Rahmen der Medizintechnik zur diagnostischen Bildgebung eingesetzt. Mit der Vielzahl diagnostischer Aufgaben steigt auch die Komplexität der Einstellung der Bildaufnahmeeinrichtungen. Entsprechend weisen Bildaufnahmeeinrichtungen eine Vielzahl an Einstellmöglichkeiten auf, das bedeutet, für eine Vielzahl von Aufnahmeparametern, die einen Aufnahmevorgang definieren, können unterschiedliche Werte festgelegt und/oder benutzerseitig verändert werden. Die Gesamtheit aller Aufnahmeparameter, die die bildgebende Messung beeinflussen beziehungsweise konfigurieren, kann dabei in einer Speichereinrichtung der Bildaufnahmeeinrichtung als ein Aufnahmeprotokoll (Aufnahmeparametersatz) abgespeichert werden. Dabei können bestimmte Aufnahmeprotokolle bereits herstellerseitig den Benutzern bereitgestellt werden, üblicherweise weisen Bildaufnahmeeinrichtungen jedoch auch Eingabeeinrichtungen auf, über die benutzerseitig durch Einstellung von Werten für die Aufnahmeparameter Aufnahmeprotokolle definiert und in der Speichereinrichtung abgespeichert werden können.

Bildaufnahmeeinrichtungen können sowohl bezüglich der Hardware als auch bezüglich der Software aktualisiert und/oder erweitert werden. Beispielsweise kann eine neue Hardwarekomponente hinzugefügt werden, die neue Aufnahmeparameter erschließt und/oder den möglichen Einstellungsraum für bestehende Aufnahmeparameter erweitert. Entsprechend können Softwarekomponenten, beispielsweise als Softwarepaket, hinzugefügt und/oder aktualisiert werden. Insgesamt kann die Gesamtheit der aktuell verfügbaren Hardwarekomponenten sowie die Versionen der aktuell verfügbaren Softwarekomponenten als aktuelle Ausstattungsversion der Bildaufnahmeeinrichtung bezeichnet werden. Aktualisierungen bezüglich der Softwarekomponenten umfassen dabei nicht nur aktuellere Versionen bereits zuvor vorhandener Softwarekomponenten, sondern auch das Erwerben neuer Lizenzen und somit den Zugriff auf neue Softwarekomponenten.

Ändert sich die Ausstattungsversion, kann es vorkommen, dass aktuell vorliegende und in der Speichereinrichtung gespeicherte Aufnahmeprotokolle nicht mehr lauffähig sind, da keine Konsistenz mit der aktuellen Ausstattungsversion gegeben ist. Beispielsweise ist es denkbar, dass bestimmte Werte für Aufnahmeparameter oder gar Aufnahmeparameter als solche nicht mehr verfügbar sind. Auch Kombinationen von Werten für Aufnahmeparameter können gegebenenfalls in einer aktuelleren Ausstattungsversion nicht länger zulässig sein. Daher ist es bekannt, eine Konsistenzüberprüfung für in der Speichereinrichtung vorliegende Aufnahmeprotokolle durchzuführen, in der diese für die neue, aktuelle Ausstattungsversion konvertiert werden können, insbesondere, indem nicht mehr existente Einstellmöglichkeiten entfernt werden und/oder nicht mehr zulässige Aufnahmeparameterkombinationen entfernt werden und/oder eine Umrechnung auf ein neues Datenformat bezüglich von Aufnahmeparametern, bei denen sich dieses geändert hat, stattfinden kann. So können die Aufnahmeprotokolle auch in der aktuellen Ausstattungsversion weiter genutzt werden. Falls ein Aufnahmeprotokoll konsistent ist, ist es selbstverständlich überhaupt nicht notwendig, etwas zu ändern.

Analog kann vorgegangen werden, wenn ein Aufnahmeprotokoll von einer anderen Bildaufnahmeeinrichtung, beispielsweise über eine Servereinrichtung beziehungsweise ein Internetportal, über das Aufnahmeprotokolle ausgetauscht werden, empfangen wird. Derartige empfangene Aufnahmeprotokolle, die für eine andere Ausstattungsversion definiert sind, können ebenso durch die Konsistenzüberprüfung zur Herstellung der Kompatibilität bearbeitet werden.

Bei der Änderung der Ausstattungsversion kann es jedoch sowohl bei einer Erweiterung des möglichen Einstellungsraums als auch bei einer Reduzierung des möglichen Einstellungsraums vorkommen, dass sich andere Kombinationen von Werten für Aufnahmeparameter beziehungsweise insgesamt neue Werte für Aufnahmeparameter als günstiger im Hinblick auf die zu erreichenden Aufnahmeziele erweisen. Möchte ein Benutzer also neue Aufnahmeparameter und/oder Optionen für Aufnahmeparameter verwenden, muss er jedes insbesondere selbst definierte Aufnahmeprotokoll in seiner Protokolldatenbank manuell anpassen. Da in Speichereinrichtungen oftmals mehrere tausend Protokolle abgespeichert werden können, ist ein enorm großer manueller Aufwand nötig. Dieser manuelle Aufwand fällt auch an, wenn ein für eine andere Ausstattungsversion definiertes Aufnahmeprotokoll, beispielsweise über ein Internetportal, heruntergeladen und in die Speichereinrichtung eingespeichert wird.

Zwar wurde im Stand der Technik bereits vorgeschlagen, herstellerseitig vorgegebene Aufnahmeprotokolle bereits seitens des Herstellers anzupassen und bei einer Aktualisierung der Ausstattungsversion der Bildaufnahmeeinrichtung mitzuliefern und/oder wenigstens bereitzustellen. Dies reduziert jedoch den manuell durchzuführenden Aufwand für benutzerseitig definierte, in der Speichereinrichtung individuell gespeicherte Aufnahmeprotokolle nicht.

Der Erfindung liegt daher die Aufgabe zugrunde, eine aufwandsarm umzusetzende Möglichkeit zur Verbesserung der Qualität von Bilddaten und/oder der Aufnahme von Bilddaten durch Optimierung von Aufnahmeprotokollen anzugeben.

Zur Lösung dieser Aufgabe ist bei einem Verfahren der eingangs genannten Art erfindungsgemäß vorgesehen, dass bei einer den möglichen Einstellungsraum für Aufnahmeparameter verändernden Aktualisierung wenigstens einer Software- und/oder Hardwarekomponente der Bildaufnahmeeinrichtung auf eine aktuelle Ausstattungsversion und/oder bei Eingang eines nicht für die aktuelle Ausstattungsversion definierten Aufnahmeprotokolls ein wenigstens eine Anpassungsregel für wenigstens einen Aufnahmeparameter, die bei Anwendbarkeit wenigstens ein Qualitätsmaß für mittels des Aufnahmeprotokolls, auf das die Anpassungsregel angewendet wird, aufgenommene Magnetresonanzdaten erhöht, umfassender Regelsatz auf alle nicht für die aktuelle Ausstattungsversion definierten Aufnahmeprotokolle angewendet wird.

Erfindungsgemäß wird also vorgeschlagen, einen seitens einer Steuereinrichtung der Bildaufnahmeeinrichtung vorliegenden Regelsatz auf nicht für die aktuelle Ausstattungsversion definierte Aufnahmeprotokolle anzuwenden, um diese hinsichtlich des Qualitätsmaßes optimieren zu können. Es wird also eine Optimierung der Aufnahmeprotokolle anhand eines regelbasierten Systems vorgeschlagen. Es ist ein Regelsatz definiert, mit dessen Anpassungsregeln die Werte von Aufnahmeparametern analysiert und gegebenenfalls optimiert werden können. Auf diese Weise ist mithin eine automatische Anpassung wenigstens von benutzerseitig erstellten und/oder importierten Aufnahmeprotokollen auf eine aktuelle Ausstattungsversion möglich, so dass eine manuelle Anpassung vermieden wird. Mögliche Optimierungen werden systemseitig bereitgestellt. In dem Regelsatz wird das Wissen über die bestmögliche Bildqualität beziehungsweise Aufnahmequalität durch die Anpassungsregeln abgebildet, die bei der Optimierung der Aufnahmeprotokolle zur Anwendung kommen.

Dies ist sowohl nach einem Hardware- und/oder Software-Update, insbesondere auch einer Lizenzänderung, vorteilhaft als auch im Rahmen der Importierung von Aufnahmeprotokollen von Bildaufnahmeeinrichtungen unterschiedlicher Ausstattungsversion. Nachdem beispielsweise Kliniken und/oder Radiologie-Praxen tendenziell immer größer werden und somit mehrere bildgebende Bildaufnahmeeinrichtungen aufweisen können, wird der Austausch von Aufnahmeprotokollen zwischen einzelnen Bildaufnahmeeinrichtungen immer wichtiger werden. Ohne eine optimale Anpassung an die Ausstattungsversion der importierenden Bildaufnahmeeinrichtung würde jedoch der Austausch nur wenig Nutzen für die entsprechende medizintechnische Einrichtung bringen, da ansonsten noch sehr viel manuelle Arbeit notwendig wäre. Selbstverständlich kann sich ein Austausch von Aufnahmeprotokollen auch auf einrichtungsübergreifende Austauschplattformen beziehen, beispielsweise Internetplattformen.

Auch wenn sich viele der hier dargestellten Beispiele auf eine Magnetresonanzeinrichtung als Bildaufnahmeeinrichtung beziehen, bei der die Abhängigkeiten zwischen Aufnahmeparametern und die Komplexität der Programmierung von Aufnahmevorgängen im Allgemeinen sehr hoch ist, mithin das erfindungsgemäße Verfahren besonders vorteilhaft angewendet werden kann, lässt es sich selbstverständlich auch auf andere Bildaufnahmeeinrichtungen anwenden, die eine Vielzahl von Aufnahmeprotokollen verwenden können, beispielsweise Computertomographieeinrichtungen.

In einer zweckmäßigen Weiterbildung der vorliegenden Erfindung kann vorgesehen sein, dass vor der Anwendung des Regelsatzes ein die Kompatibilität der nicht für die aktuelle Ausstattungsversion definierten Aufnahmeprotokolle mit der aktuellen Ausstattungsversion herstellender Konversionsalgorithmus angewendet wird. Es wird mithin zunächst eine Konsistenzüberprüfung durchgeführt, wobei bei nicht mit der aktuellen Ausstattungsversion konsistenten Aufnahmeprotokollen zunächst eine Konvertierung stattfindet, die die Kompatibilität mit der aktuellen Ausstattungsversion herstellt, ohne allerdings Optimierungen hinsichtlich des Qualitätsmaßes vorzunehmen. Beispielsweise kann im Rahmen der Herstellung der Kompatibilität eine Entfernung von nichtmehr existenten Einstellmöglichkeiten und/oder nicht mehr zulässigen Aufnahmeparameterkombinationen und/oder eine Umrechnung auf ein neues Datenformat erfolgen. An die Konsistenzüberprüfung und gegebenenfalls Konsistenzherstellung kann, gegebenenfalls benutzerseitig wählbar, dann eine Optimierung unter Verwendung des Regelsatzes anschließen.

Eine zweckmäßige Weiterbildung der vorliegenden Erfindung sieht vor, dass als Qualitätsmaß ein die Bildqualität und/oder eine Reduzierung der Aufnahmedauer bei wenigstens gleichbleibender Bildqualität beschreibendes Maß verwendet wird. Dabei ist es bevorzugt, ein auf die Bildqualität unmittelbar bezogenes Maß anzusetzen, wobei entsprechende Qualitätsmaße im Stande der Technik bereits bekannt sind und sich je nach Aufnahmeziel des zu optimierenden Aufnahmeprotokolls auch unterscheiden können, beispielsweise auf eine gute Erkennbarkeit von Strukturen (Kontrast) bezogene Maße genauso verwendet werden können wie auf eine gute Homogenität abzielende Maße, Rauschanteile auswertende Maße und dergleichen.

In einer zweckmäßigen Weiterbildung der Erfindung ist vorgesehen, dass aufgrund einer Anpassungsregel anzupassende Aufnahmeparameter eines anzupassenden Aufnahmeprotokolls zunächst einem Benutzer als Vorschlag zur Anzeige gebracht werden und erst nach einer bestätigenden Benutzereingabe auf die vorgeschlagenen Werte angepasst werden. Auf diese Weise kann sich der Benutzer also mögliche Optimierungen von der Bildaufnahmeeinrichtung, konkret einer Steuereinrichtung der Bildaufnahmeeinrichtung, berechnen lassen und diese nach Überprüfung bestätigen, falls er keine vollständig automatische Einstellung wünscht. Die neuen Werte der Aufnahmeparameter werden dem Benutzer mithin präsentiert, so dass er diese bestätigen oder ablehnen kann. Mit besonderem Vorteil kann zusätzlich eine Anzeige einer Begründungsinformation erfolgen, beispielsweise umfassend eine Differenz des Qualitätsmaßes mit und ohne Anpassung und/oder eine in der Anpassungsregel hinterlegte Motivationsinformation. Auf diese Weise wird es dem Benutzer vereinfacht, die Hintergründe des Vorschlags nachzuvollziehen.

Vorzugsweise können die in der Speichereinrichtung zu speichernden Aufnahmeprotokolle herstellerseitig vorgegebene Aufnahmeprotokolle umfassen, wobei bei einer den möglichen Einstellungsraum für Aufnahmeparameter verändernden Aktualisierung wenigstens einer Software- und/oder Hardwarekomponente der Bildaufnahmeeinrichtung auf eine aktuelle Ausstattungsversion die herstellerseitig vorgegebenen Benutzerprotokolle mit aktualisiert werden. Das bedeutet, mit der Aktualisierung mit der Software- und/oder Hardwarekomponente werden herstellerseitig auch bereits angepasste und optimierte Aufnahmeprotokolle geliefert, die mithin weder konvertiert noch weiter optimiert werden müssen, sondern die durch die Aktualisierung gegebenen Möglichkeiten bereits optimal ausnutzen. Auf diese Weise wird der Aufwand seitens der Bildaufnahmeeinrichtung reduziert.

Wie bereits erwähnt, kann auch vorgesehen sein, dass die Aktualisierung eine Veränderung einer Lizenz für Softwarekomponenten umfasst, durch die Einstellmöglichkeiten hinzugefügt werden und/oder wegfallen. Auf eine derartige Lizenzänderung kann mithin auch durch Optimierung zumindest der benutzerseitig definierten Aufnahmeprotokolle reagiert werden, so dass, unabhängig davon, ob Einstellmöglichkeiten hinzukommen oder wegfallen, für den Benutzer immer die beste Bildqualität zur Verfügung gestellt wird.

In konkreter Ausgestaltung kann eine Anpassungsregel wenigstens eine zur Anwendung der Anpassungsregel zu erfüllende Bedingung und eine bei Erfüllung der wenigstens einen Bedingung durchzuführende Anpassungsmaßnahme umfassen. Dabei kann die wenigstens eine Bedingung die aktuelle Ausstattungsversion und/oder wenigstens einen der Aufnahmeparameter auswerten. Bedingungen können mithin beispielsweise logische Abfragen auf bestimmte Informationen umfassen. So kann überprüft werden, ob ein aktueller Wert eines Aufnahmeparameters größer oder kleiner einer Konstante ist, genau einer Konstante entspricht und/oder dieser Konstante nicht entspricht, was sich selbstverständlich auch auf Kombinationen von Aufnahmeparametern übertragen lässt. Es kann ferner auch bezüglich von Softwarekomponenten überprüft werden, ob bestimmte Lizenzen verfügbar sind und/oder in Bezug auf Softwarekomponenten und Hardwarekomponenten, ob die entsprechende Komponente tatsächlich vorliegt und funktionsfähig ist. Anpassungsmaßnahmen beinhalten den anzupassenden Aufnahmeparameter und den neuen Wert, welcher im Übrigen nicht zwangsläufig eine Konstante sein muss, sondern sich auch beispielsweise in Abhängigkeit von anderen Aufnahmeparametern ergeben kann.

Bei einer Optimierung eines Aufnahmeprotokolls wird nun versucht, alle Anpassungsregeln des Regelsatzes anzuwenden. Falls die Bedingungen einer Anpassungsregel zutreffen, werden die entsprechenden Anpassungsmaßnahmen durchgeführt, mithin der wenigstens eine anzupassende Aufnahmeparameter geändert und somit optimiert. Hieraus folgt aber auch, dass, je größer die Regelbasis ist, über die die Bildaufnahmeeinrichtung verfügt, desto wahrscheinlicher ein Aufnahmeprotokoll auch optimiert werden kann.

In einem konkreten Beispiel kann beispielsweise eine Softwareaktualisierung stattgefunden haben, bei der ein Aufnahmeparameter A bisher zwei verschiedene Konfigurationsmöglichkeiten (Werte) A1 und A2 aufweisen konnte. Mit einer neuen Softwareversion wird eine neue Konfigurationsmöglichkeit A3 eingeführt, die bessere Bildqualität verspricht, falls der Aufnahmeparameter B den Wert B1 besitzt. Eine Regel für die Optimierung könnte dann beispielsweise die Bedingung umfassen, dass der Aufnahmeparameter B == B1 sein soll und die Anpassungsmaßnahme könnte umfassen, dass der Aufnahmeparameter A = A3 gesetzt wird. Dabei sei darauf hingewiesen, dass die bisher eingesetzte Konsistenzüberprüfung den Parameter A nicht verändern würde, da der Wert A2 weiterhin Gültigkeit besitzt.

Bei einem anderen Beispiel hat ein Benutzer eine erweiterte Lizenz für Software einer Magnetresonanzeinrichtung erworben. Dabei hat der Aufnahmeparameter A drei verschiedene mögliche Werte A1, A2, A3. A3 ist allerdings mit der Lizenz L1 versehen, die der Benutzer erworben hat, nachdem die Verwendung des Wertes A3 eine bessere Bildqualität verspricht. Bei einer Optimierung von für Ausstattungsversionen ohne Lizenz definierten Aufnahmeprotokollen kann eine Anpassungsregel mithin die Bedingung umfassen, dass die Lizenz L1 verfügbar ist, woraufhin als Anpassungsmaßnahme der Parameter A = A3 gesetzt wird.

Eine allgemeine zweckmäßige Weiterbildung der vorliegenden Erfindung sieht vor, dass der Regelsatz bei einer Aktualisierung wenigstens teilweise mit der aktualisierten Komponente und/oder wenigstens teilweise unabhängig von dieser erhalten wird. Während es zweckmäßig ist, beispielsweise seitens des Herstellers den Regelsatz bereits mitzuliefern, wenn eine Aktualisierung auf eine neue Ausstattungsversion stattfindet, ist es auch denkbar und vorteilhaft, Regelsätze insgesamt importierbar und/oder exportierbar zu gestalten, so dass Regelsätze auch nachgeliefert werden können, beispielsweise vom Hersteller oder aber auch von speziellen Applikationsspezialisten, die ihr Wissen über die optimale Nutzung von Einstellmöglichkeiten über den Regelsatz bereitstellen können.

Dabei sei allgemein auch darauf hingewiesen, dass der Regelsatz in einer domänenspezifischen Regelsprache in der Steuereinrichtung und/oder der Speichereinrichtung hinterlegt werden kann, wenn eine solche domänenspezifische Regelsprache (DSL - domain specific language) vorliegt.

In einer Weiterbildung kann wenigstens eines der nicht für die aktuelle Ausstattungsversion definierten Aufnahmeprotokolle und/oder wenigstens ein Teil der Regelsätze über eine Kommunikationsverbindung, insbesondere von einer Servereinrichtung, empfangen werden. Bezüglich des Austauschs von Aufnahmeprotokollen zwischen unterschiedlichen Bildaufnahmeeinrichtungen und/oder unterschiedlichen Benutzern wurde bereits ausgeführt, wobei sich bei der Servereinrichtung hier beispielsweise um ein Internetportal und/oder ein einer medizinischen Einrichtung, insbesondere einer Klinik und/oder einer Radiologie-Praxis, zugeordnetes Portal handeln kann. Auch bezüglich der Regelsätze ist ein Import und Export wie dargelegt wurde, möglich und sinnvoll.

Vorzugsweise können die Anpassungsregeln des Regelsatzes aufgrund einer Benutzereingabe definiert werden. Beispielsweise können die Anpassungsregeln von Applikationsspezialisten und/oder Mitarbeitern des Herstellers, die herstellerseitig vorgegebene Aufnahmeprotokolle aktualisieren, definiert werden. In beiden Fällen wird das Wissen über die bestmögliche erreichbare Bildqualität in die Anpassungsregel umgesetzt und somit auch für insbesondere benutzerseitig definierte Aufnahmeprotokolle bereitgestellt, welche automatisch im Hinblick auf das Qualitätsmaß verbessert werden können.

Denkbar ist es in einer Ausgestaltung der vorliegenden Erfindung jedoch auch, dass wenigstens eine Anpassungsregel des Regelsatzes durch einen Algorithmus der künstlichen Intelligenz mittels Maschinenlernens und/oder durch eine Auswertung von durch Simulation und/oder Durchführung wenigstens einer Testmessung mit in der Anpassungsregel relevanten Aufnahmeparametern ermittelten Messdaten ermittelt wird. Es ist also auch denkbar, Anpassungsregeln wenigstens teilweise automatisch zu ermitteln, indem auf Methoden der künstlichen Intelligenz und/oder auf die Auswertung von Messungen und/oder Simulationen im Hinblick auf das Qualitätsmaß zurückgegriffen wird. Für Techniken der künstlichen Intelligenz, beispielsweise künstliche neuronale Netze, können beispielsweise Trainingsdaten bereitgestellt werden, die zu verschiedenen Kombinationen von Aufnahmeparametern Informationen zum Qualitätsmaß erhalten und Zusammenhänge zwischen Aufnahmeparametern herstellen, die zur Optimierung der Bildqualität führen können.

Neben dem Verfahren betrifft die vorliegende Erfindung auch eine Bildaufnahmeeinrichtung, insbesondere eine Magnetresonanzeinrichtung, aufweisend eine Speichereinrichtung für eine Mehrzahl von Aufnahmeparameter der Bildaufnahmeeinrichtung enthaltenden, einen Aufnahmevorgang definierenden Aufnahmeprotokollen und eine Steuereinrichtung, die zur Durchführung des erfindungsgemäßen Verfahrens ausgebildet ist. Sämtliche Ausführungen bezüglich des erfindungsgemäßen Verfahrens lassen sich analog auf die erfindungsgemäße Bildaufnahmeeinrichtung übertragen, mit welcher mithin ebenso die bereits genannten Vorteile erhalten werden können.

Ein erfindungsgemäßes Computerprogramm ist beispielsweise direkt in einen Speicher einer Steuereinrichtung einer Bildaufnahmeeinrichtung ladbar und weist Programmmittel auf, um die Schritte eines hierin beschriebenen Verfahrens auszuführen, wenn das Computerprogramm in der Steuereinrichtung der Bildaufnahmeeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein genanntes Computerprogramm umfassen und insbesondere derart ausgestaltet sind, dass sie bei Verwendung des Datenträgers in einer Steuereinrichtung einer Bildaufnahmeeinrichtung ein erfindungsgemäßes Verfahren durchführen. Bei dem erfindungsgemäßen elektronisch lesbaren Datenträger handelt es sich bevorzugt um einen nichttransienten Datenträger, beispielsweise eine CD-ROM.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens, und
- Fig. 2: eine erfindungsgemäße Magnetresonanzeinrichtung.

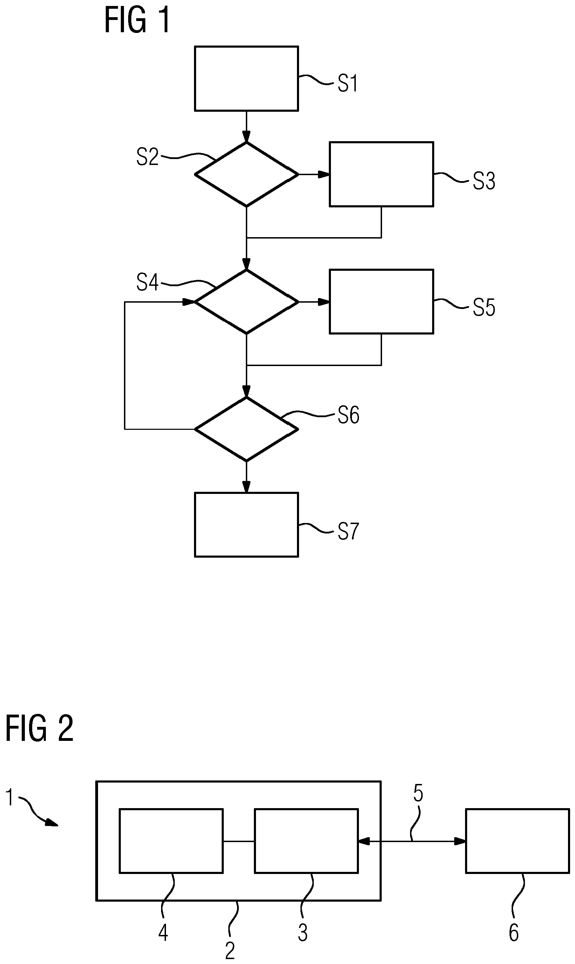

Fig. 1 zeigt einen generellen Ablaufplan eines Ausführungsbeispiels des erfindungsgemäßen Verfahrens. Dabei tritt in einem Schritt S1 ein Triggerereignis ein, das zur (falls notwendig) Konvertierung und (falls möglich) Optimierung bestimmter Aufnahmeprotokolle an einer Bildaufnahmeeinrichtung, hier einer Magnetresonanzeinrichtung, führt. Die Bildaufnahmeeinrichtung weist dabei eine Speichereinrichtung auf, in der Aufnahmeprotokolle gespeichert werden können. Dabei existieren zwei Arten von Aufnahmeprotokollen, nämlich zum einen herstellerseitig vorgegebene Aufnahmeprotokolle, zum anderen benutzerseitig definierte Aufnahmeprotokolle. Herstellerseitig vorgegebene Aufnahmeprotokolle werden vom Hersteller für verschiedene mögliche Ausstattungsversionen der Magnetresonanzeinrichtung, was Hardware- und Softwarekomponenten angeht, bereitgestellt, wobei die Ausstattung vorliegend auch Softwarelizenzen umfassen soll.

Ist das Triggerereignis mithin eine Aktualisierung der Magnetresonanzeinrichtung im Hinblick auf wenigstens eine Softwarekomponente und/oder wenigstens eine Hardwarekomponente, umfassen auf Konvertierung und Optimierung zu überprüfende Aufnahmeprotokolle die in der Speichereinrichtung gespeicherten, benutzerseitig definierten Aufnahmeprotokolle, die für eine von der aktuellen Ausstattungsversion abweichende Ausstattungsversion definiert sind. Ein Triggerereignis kann jedoch auch gegeben sein, wenn ein für eine andere Ausstattungsversion als die aktuelle Ausstattungsversion der Magnetresonanzeinrichtung definiertes Aufnahmeprotokoll importiert wird, beispielsweise von einer anderen Magnetresonanzeinrichtung und/oder über ein entsprechendes Austauschportal. Dann ist dieses importierte Aufnahmeprotokoll auf Konsistenz und/oder Optimierungsmöglichkeiten zu überprüfen.

In einem Schritt S2 des Verfahrens wird überprüft, ob das Aufnahmeprotokoll konsistent mit der aktuellen Ausstattungsversion ist. Ist dies nicht der Fall, wird in einem Schritt S3 ein Konversionsalgorithmus ausgeführt, der die Konsistenz herstellt, mithin nicht mehr zulässige Einstellmöglichkeiten und/oder Aufnahmeparameterkombinationen entfernt und/oder geänderte Datenformate anpasst.

Nach der gegebenenfalls durchgeführten Konvertierung zur Konsistenz mit der aktuellen Ausstattungsversion wird das Aufnahmeprotokoll gegenüber verschiedenen Anpassungsregeln eines Regelsatzes überprüft. Die Anpassungsregeln sind auf die aktuelle Ausstattungsversion angepasst, mithin ausstattungsversionsspezifisch, und können bei Aktualisierung hinsichtlich von Software- und/oder Hardwarekomponenten bereits mitgeliefert werden, oder aber auch allgemein importierbar und exportierbar sein, mithin wenigstens teilweise nachgeliefert werden und/oder dediziert von Applikationsspezialisten bereitgestellt werden. Anpassungsregeln können wenigstens teilweise auf Benutzereingaben basieren und/oder wenigstens teilweise durch Methoden der künstlichen Intelligenz und/oder Auswertung von Messergebnissen ermittelt werden, zielen aber in jedem Fall darauf ab, ein durch den Aufnahmevorgang, der durch das Aufnahmeprotokoll beschrieben wird, erhaltenes Qualitätsmaß für die Bilddaten zu erhöhen, das vorliegend auf die Bildqualität der Bilddaten, hier Magnetresonanzdaten, abzielt.

Anpassungsregeln umfassen wenigstens eine Bedingung und wenigstens eine Anpassungsmaßnahme, wobei bei Erfüllung der Bedingung, die die Ausstattungsversion und/oder Aufnahmeparameter des Aufnahmeprotokolls auswerten kann, die wenigstens eine Anpassungsmaßnahme, die den Wert wenigstens eines Aufnahmeparameters anpasst (oder gegebenenfalls hinzufügt), durchgeführt wird. Die Überprüfung der Bedingungen findet in einem Schritt S4 statt, die Durchführung der Anpassungsmaßnahme bei Erfüllung der wenigstens einen Bedingung in einem Schritt S5.

In einem Schritt S6 wird überprüft, ob weitere Anpassungsregeln in dem Regelsatz vorliegen. Ist dies der Fall, wird erneut der Schritt S4 und gegebenenfalls der Schritt S5 für die nächste Anpassungsregel durchgeführt; sind alle Anpassungsregeln des Regelsatzes bearbeitet, endet das Verfahren in einem schritt S7, in dem mithin nicht nur mit der aktuellen Ausstattungsversion konsistente, sondern auch bezüglich der Bildqualität optimierte Aufnahmeprotokolle in der Speichereinrichtung der Magnetresonanzeinrichtung vorliegen.

In dem Schritt S5 kann in einer Variante des erfindungsgemäßen Verfahrens auch vorgesehen sein, dass die aus der Anpassungsregel resultierenden neuen Werte für die Aufnahmeparameter zunächst einem Benutzer zur Anzeige gebracht werden, wonach dieser die Anpassung zunächst noch bestätigen muss. Dies ist jedoch optional und kann beispielsweise als Einstellung vom Benutzer gewählt werden. Zweckmäßig werden bei einer solchen Anzeige zusätzlich weitere Informationen dargestellt, die die Änderung für den Benutzer motivieren.

Fig. 2 zeigt eine Prinzipskizze einer Bildaufnahmeeinrichtung 1, die vorliegend als Magnetresonanzeinrichtung 2 ausgebildet ist, wobei der Übersichtlichkeit halber übliche, grundsätzlich bekannte Komponenten der Bildaufnahmeeinrichtung 1 ausgespart sind und lediglich die für die vorliegende Erfindung relevanten Komponenten dargestellt sind. Der Betrieb der Bildaufnahmeeinrichtung 1 wird mittels einer Steuereinrichtung 3 gesteuert, der eine Speichereinrichtung 4 zugeordnet ist, in der Aufnahmeprotokolle und gegebenenfalls auch der Regelsatz abgelegt werden können. Die Steuereinrichtung 3 ist zur Durchführung des erfindungsgemäßen Verfahrens ausgebildet.

Über eine Kommunikationsverbindung 5 kann die Steuereinrichtung 3 mit wenigstens einer Servereinrichtung 6 kommunizieren, von der beispielsweise Aufnahmeprotokolle, Softwareupdates, Regelsätze und dergleichen abgerufen werden können.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zum Betrieb einer Bildaufnahmeeinrichtung (1), insbesondere einer Magnetresonanzeinrichtung (2), welche eine Speichereinrichtung (4) für eine Mehrzahl von Aufnahmeparameter der Bildaufnahmeeinrichtung (1) enthaltenden, einen Aufnahmevorgang definierenden Aufnahmeprotokollen aufweist, **dadurch gekennzeichnet, dass** bei einer den möglichen Einstellungsraum für Aufnahmeparameter verändernden Aktualisierung wenigstens einer Software- und/oder Hardwarekomponente der Bildaufnahmeeinrichtung (1) auf eine aktuelle Ausstattungsversion und/oder bei Eingang eines nicht für die aktuelle Ausstattungsversion definierten Aufnahmeprotokolls ein wenigstens eine Anpassungsregel für wenigstens einen Aufnahmeparameter, die bei Anwendbarkeit wenigstens ein Qualitätsmaß für mittels des Aufnahmeprotokolls, auf das die Anpassungsregel angewendet wird, aufgenommene Bilddaten erhöht, umfassender Regelsatz auf alle nicht für die aktuelle Ausstattungsversion definierten Protokolle angewendet wird.

2. Verfahren nach Anspruch 1, **dadurch gekennzeichnet, dass** vor der Anwendung des Regelsatzes ein die Kompatibilität der nicht für die aktuelle Ausstattungsversion definierten Aufnahmeprotokolle mit der aktuellen Ausstattungsversion herstellender Konversionsalgorithmus angewendet wird.

3. Verfahren nach Anspruch 1 oder 2, **dadurch gekennzeichnet, dass** als Qualitätsmaß ein die Bildqualität und/oder eine Reduzierung der Aufnahmedauer bei wenigstens gleichbleibender Bildqualität beschreibendes Maß verwendet wird.

4. Verfahren nach einem der vorangehenden Ansprüche, **dadurch gekennzeichnet, dass** aufgrund einer Anpassungsregel anzupassende Aufnahmeparameter eines anzupassenden Aufnahmeprotokolls zunächst einem Benutzer als Vorschlag zur Anzeige gebracht werden und erst nach einer bestätigenden Benutzereingabe auf die vorgeschlagenen Werte angepasst werden.

5. Verfahren nach einem der vorangehenden Ansprüche, **dadurch gekennzeichnet, dass** die in der Speichereinrichtung (4) zu speichernden Aufnahmeprotokolle herstellerseitig vorgegebene Aufnahmeprotokolle umfassen, wobei bei einer den möglichen Einstellungsraum für Aufnahmeparameter verändernden Aktualisierung wenigstens einer Software- und/oder Hardwarekomponente der Bildaufnahmeeinrichtung (1) auf eine aktuelle Ausstattungsversion die herstellerseitig vorgegebenen Aufnahmeprotokolle mit aktualisiert werden.

6. Verfahren nach einem der vorangehenden Ansprüche, **dadurch gekennzeichnet, dass** die Aktualisierung eine Veränderung einer Lizenz für Softwarekomponenten umfasst, durch die Einstellmöglichkeiten hinzugefügt werden und/oder wegfallen.

7. Verfahren nach einem der vorangehenden Ansprüche, **dadurch gekennzeichnet, dass** eine Anpassungsregel wenigstens eine zur Anwendung der Anpassungsregel zu erfüllende Bedingung und eine bei Erfüllung der wenigstens einen Bedingung durchzuführende Anpassungsmaßnahme umfasst.

8. Verfahren nach Anspruch 7, **dadurch gekennzeichnet, dass** die Bedingung die aktuelle Ausstattungsversion und/oder wenigstens einen der Aufnahmeparameter auswertet.

9. Verfahren nach einem der vorangehenden Ansprüche, **dadurch gekennzeichnet, dass** der Regelsatz bei einer Aktualisierung wenigstens teilweise mit der aktualisierten Komponente und/oder wenigstens teilweise unabhängig von dieser erhalten wird.

10. Verfahren nach einem der vorangehenden Ansprüche, **dadurch gekennzeichnet, dass** wenigstens eines der nicht für die aktuelle Ausstattungsversion definierten Aufnahmeprotokolle und/oder wenigstens ein Teil des Regelsatzes über eine Kommunikationsverbindung (5), insbesondere von einer Servereinrichtung (6), empfangen wird.

11. Verfahren nach einem der vorangehenden Ansprüche, **dadurch gekennzeichnet, dass** wenigstens eine Anpassungsregel des Regelsatzes durch einen Algorithmus der künstlichen Intelligenz mittels Maschinenlernen und/oder durch eine Auswertung von durch Simulation und/oder Durchführung wenigstens einer Testmessung mit in der Anpassungsregel relevanten Aufnahmeparametern ermittelten Messdaten ermittelt wird.

12. Bildaufnahmeeinrichtung (1), aufweisend eine Speichereinrichtung (4) für eine Mehrzahl von Aufnahmeparameter der Bildaufnahmeeinrichtung (1) enthaltenden, einen Aufnahmevorgang definierenden Aufnahmeprotokollen und eine Steuereinrichtung (3), die zur Durchführung eines Verfahrens nach einem der vorangehenden Ansprüche ausgebildet ist.

13. Computerprogramm, welches die Schritte eines Verfahrens nach einem der Ansprüche 1 bis 10 durchführt, wenn es auf einer Recheneinrichtung ausgeführt wird.

14. Elektronisch lesbarer Datenträger, auf dem ein Computerprogramm nach Anspruch 12 gespeichert ist.

## Geänderte Patentansprüche

### Geänderte Patentansprüche gemäss Regel 137(2) EPÜ.

1. Verfahren zum Betrieb einer Bildaufnahmeeinrichtung (1), insbesondere einer Magnetresonanzeinrichtung (2), welche eine Speichereinrichtung (4) für eine Mehrzahl von Aufnahmeparameter der Bildaufnahmeeinrichtung (1) enthaltenden, einen Aufnahmevorgang definierenden Aufnahmeprotokollen aufweist, **dadurch gekennzeichnet, dass** bei einer den möglichen Einstellungsraum für Aufnahmeparameter verändernden Aktualisierung wenigstens einer Software- und/oder Hardwarekomponente der Bildaufnahmeeinrichtung (1) auf eine aktuelle Ausstattungsversion und/oder bei Eingang eines nicht für die aktuelle Ausstattungsversion definierten Aufnahmeprotokolls ein Regelsatz auf alle nicht für die aktuelle Ausstattungsversion definierten Protokolle angewendet wird, welcher wenigstens eine Anpassungsregel für wenigstens einen Aufnahmeparameter umfasst, wobei die Anpassungsregel bei Anwendbarkeit wenigstens ein Qualitätsmaß für mittels des Aufnahmeprotokolls, auf das die Anpassungsregel angewendet wird, aufgenommene Bilddaten erhöht.

2. Verfahren nach Anspruch 1, **dadurch gekennzeichnet, dass** vor der Anwendung des Regelsatzes ein die Kompatibilität der nicht für die aktuelle Ausstattungsversion definierten Aufnahmeprotokolle mit der aktuellen Ausstattungsversion herstellender Konversionsalgorithmus angewendet wird.

3. Verfahren nach Anspruch 1 oder 2, **dadurch gekennzeichnet, dass** als Qualitätsmaß ein die Bildqualität und/oder eine Reduzierung der Aufnahmedauer bei wenigstens gleichbleibender Bildqualität beschreibendes Maß verwendet wird.

4. Verfahren nach einem der vorangehenden Ansprüche, **dadurch gekennzeichnet, dass** aufgrund einer Anpassungsregel anzupassende Aufnahmeparameter eines anzupassenden Aufnahmeprotokolls zunächst einem Benutzer als Vorschlag zur Anzeige gebracht werden und erst nach einer bestätigenden Benutzereingabe auf die vorgeschlagenen Werte angepasst werden.

5. Verfahren nach einem der vorangehenden Ansprüche, **dadurch gekennzeichnet, dass** die in der Speichereinrichtung (4) zu speichernden Aufnahmeprotokolle herstellerseitig vorgegebene Aufnahmeprotokolle umfassen, wobei bei einer den möglichen Einstellungsraum für Aufnahmeparameter verändernden Aktualisierung wenigstens einer Software- und/oder Hardwarekomponente der Bildaufnahmeeinrichtung (1) auf eine aktuelle Ausstattungsversion die herstellerseitig vorgegebenen Aufnahmeprotokolle mit aktualisiert werden.

6. Verfahren nach einem der vorangehenden Ansprüche, **dadurch gekennzeichnet, dass** die Aktualisierung eine Veränderung einer Lizenz für Softwarekomponenten umfasst, durch die Einstellmöglichkeiten hinzugefügt werden und/oder wegfallen.

7. Verfahren nach einem der vorangehenden Ansprüche, **dadurch gekennzeichnet, dass** eine Anpassungsregel wenigstens eine zur Anwendung der Anpassungsregel zu erfüllende Bedingung und eine bei Erfüllung der wenigstens einen Bedingung durchzuführende Anpassungsmaßnahme umfasst.

8. Verfahren nach Anspruch 7, **dadurch gekennzeichnet, dass** die Bedingung die aktuelle Ausstattungsversion und/oder wenigstens einen der Aufnahmeparameter auswertet.

9. Verfahren nach einem der vorangehenden Ansprüche, **dadurch gekennzeichnet, dass** der Regelsatz bei einer Aktualisierung wenigstens teilweise mit der aktualisierten Komponente und/oder wenigstens teilweise unabhängig von dieser erhalten wird.

10. Verfahren nach einem der vorangehenden Ansprüche, **dadurch gekennzeichnet, dass** wenigstens eines der nicht für die aktuelle Ausstattungsversion definierten Aufnahmeprotokolle und/oder wenigstens ein Teil des Regelsatzes über eine Kommunikationsverbindung (5), insbesondere von einer Servereinrichtung (6), empfangen wird.

11. Verfahren nach einem der vorangehenden Ansprüche, **dadurch gekennzeichnet, dass** wenigstens eine Anpassungsregel des Regelsatzes durch einen Algorithmus der künstlichen Intelligenz mittels Maschinenlernen und/oder durch eine Auswertung von durch Simulation und/oder Durchführung wenigstens einer Testmessung mit in der Anpassungsregel relevanten Aufnahmeparametern ermittelten Messdaten ermittelt wird.

12. Bildaufnahmeeinrichtung (1), aufweisend eine Speichereinrichtung (4) für eine Mehrzahl von Aufnahmeparameter der Bildaufnahmeeinrichtung (1) enthaltenden, einen Aufnahmevorgang definierenden Aufnahmeprotokollen und eine Steuereinrichtung (3), die zur Durchführung eines Verfahrens nach einem der vorangehenden Ansprüche ausgebildet ist.

13. Computerprogramm, welches die Schritte eines Verfahrens nach einem der Ansprüche 1 bis 10 durchführt, wenn es auf einer Recheneinrichtung ausgeführt wird.

14. Elektronisch lesbarer Datenträger, auf dem ein Computerprogramm nach Anspruch 13 gespeichert ist.
